# EUROPEAN PATENT APPLICATION

(11) **EP 2 090 659 A1**
(43) Date of publication of application: **19.08.2009**
(21) Application number: 08002764.2
(22) Date of filing: 14.02.2008
(51) Int. Cl.: C12N 15/867, C12N 15/10

(54) **Infectious particle, process for its preparation and use thereof**

(71) Applicant: Fraunhofer-Gesellschaft zur Förderung der angewandten Forschung e.V., 80686 München (DE)
(72) Inventor: Breun, Sabine, 04105 Leipzig (DE); Baumann, Jörg, 04105 Leipzig (DE)
(74) Representative: Hartz, Nikolai

(57) **Abstract**

The present invention relates to an infectious particle having a surface displaying a ligand binding to a CD4 receptor for selectively infecting dividing CD4⁺ cells, said particle comprising:
(a) one or more structural proteins, and
(b) a vector containing a gene of interest functional in a CD4⁺ cell, said gene of interest encoding a Forkhead box protein 3 or a protein inducing expression of Forkhead box protein 3 in the CD4⁺ cell.

## Description

### Field of the Invention

The present invention relates to a specific infectious particle. Moreover, the present invention relates to a composition containing the infectious particle of the invention and the use of the infectious particle for producing antigen-specific regulatory T cells. The present invention also relates to a process for preparing antigen-specific regulatory T cells *in vitro* and a kit of parts for antigen-specific regulatory T cell induction. Finally, the present invention relates to a specific infected T cell.

### Background of the Invention

T cells represent a component of the immune system and belong to a group of white blood cells known as lymphocytes playing a central role in cell-mediated immunity. Regulatory T cells represent a sub-population of T cells, which are capable of suppressing activation of the immune system and thereby maintain immune system homeostasis and tolerance to self-antigens. Regulatory T cells, T_{Reg}s, express the immunoglobulin CD4 (CD4⁺ regulatory T cells). T_{Reg}s control an antigen-specific immune response upon activation. Specifically, T_{Reg}s prevent an immune response against a specific antigen and thereby accomplish tolerance of the immune system towards the specific antigen.

T_{Reg}s are activated by Forkhead box protein 3 [FoxP3; Nat. Rev. Immunol. (2007) 7, 305]. FoxP3 is a transcription factor that is involved in the differentiation of CD4⁺ T cells into regulatory T cells. FoxP3 is expressed in regulatory T cells which comprise 5 to 10% of a healthy individual's CD4⁺ T cell population [Science (2003) *299*, 1030]. The human FoxP3 amino acid sequence and cDNA sequence are disclosed in GenBank accession number NM_014009 (VERSION NMJH4009.2 GI:31982942). Retroviral expression of FoxP3 is sufficient to convert CD4⁺ T cells into regulatory T cells [Science (2003) 299, 1057]. FoxP3 expression can be induced by treatment of cells with transforming growth factor beta [TGF-β; Nat. Immunol. (2007) 8, 345] or certain immunosuppressive drugs [Blood (2006) 107, 1018; Blood (2007) 109, 244].

WO2007/06595 discloses T cells transiently transfected with RNA encoding FoxP3, and methods of transfecting T cells with RNA by electroporation. T_{Reg} cells comprising an exogenous RNA encoding FoxP3 are suggested for the production of a medicament for immunotherapy.

An efficient therapeutic intervention using T_{Reg}s is presently, however, not possible since an unselective activation of T_{Reg}s results in a general weakening of the immune system.

### Summary of the Invention

It is an object of the present invention to selectively activate T_{Reg}s specific for an antigen of interest in the presence of a population of T_{Reg}s having different antigen specificities while excluding activation of the T_{Reg}s having different antigen specificities.

It is an object of the invention to provide a means for selectively activating T_{Reg}s specific for an antigen of interest in the presence of further T_{Reg}s specific for different antigens in a T_{Reg} population.

It is a further object of the invention to provide a composition containing a means for selectively activating T_{Reg}s specific for an antigen of interest.

These objects are attained according to the claims. Specifically, the present invention provides an infectious particle having a surface displaying a ligand binding to a CD4 receptor and selectively infecting dividing CD4⁺ cells, said particle comprising:
(a) one or more structural proteins, and
(b) a viral vector containing a gene of interest functional in a CD4⁺ cell, said gene of interest encoding a Forkhead box protein 3 or a protein inducing expression of Forkhead box protein 3 in the CD4⁺ cell.

The present invention also provides a composition comprising:
(i) the infectious particle according to the invention, and
(ii) a carrier.

The present invention also provides a process for producing an infectious particle according to any one of claims 1 to 10, comprising an *in vitro* step of contacting one or more structural proteins with a viral vector containing a gene of interest functional in a CD4⁺ cell, said gene of interest encoding a Forkhead box protein 3 or a protein inducing expression of Forkhead box protein 3 in the CD4⁺ cell.

Furthermore, the present invention provides the use of the infectious particle according to the invention for producing antigen-specific regulatory T cells.

The present invention also provides a process for preparing antigen-specific regulatory T cells *in vitro,* which comprises:
(i) contacting antigen-presenting cells with an antigen reactive with the antigen-presenting cells for providing antigen-presenting cells complexed with an antigen,
(ii) activation of T cells with the antigen-presenting cells complexed with an antigen for providing activated T cells, and
(iii) infecting the activated T cells with an infectious particle of the invention for expressing or inducing expression of Forkhead box protein 3 so as to prepare an antigen-specific regulatory T cell.

The present invention also provides a kit of parts for antigen-specific regulatory T cell induction, which comprises a virus-like particle selectively infecting CD4⁺ cells and one or more antigens.

Finally, the present invention provides a T-cell infected with a retroviral vector containing a gene of interest functional in a CD4⁺ cell, which encodes a Forkhead box protein 3 or a protein inducing expression of Forkhead box protein 3.

The present invention is based on the concept of using an infectious particle selectively infecting actively dividing T_{Reg}s in the presence of an antigen of interest. The infectious particle contains a vector comprising a FoxP3 protein which is expressed in the infected cell whereby an activated T_{Reg} is provided which is specific for the antigen of interest. Accordingly, selective T_{Reg} mediated suppression of the immune system is accomplished.

### Description of the Figures

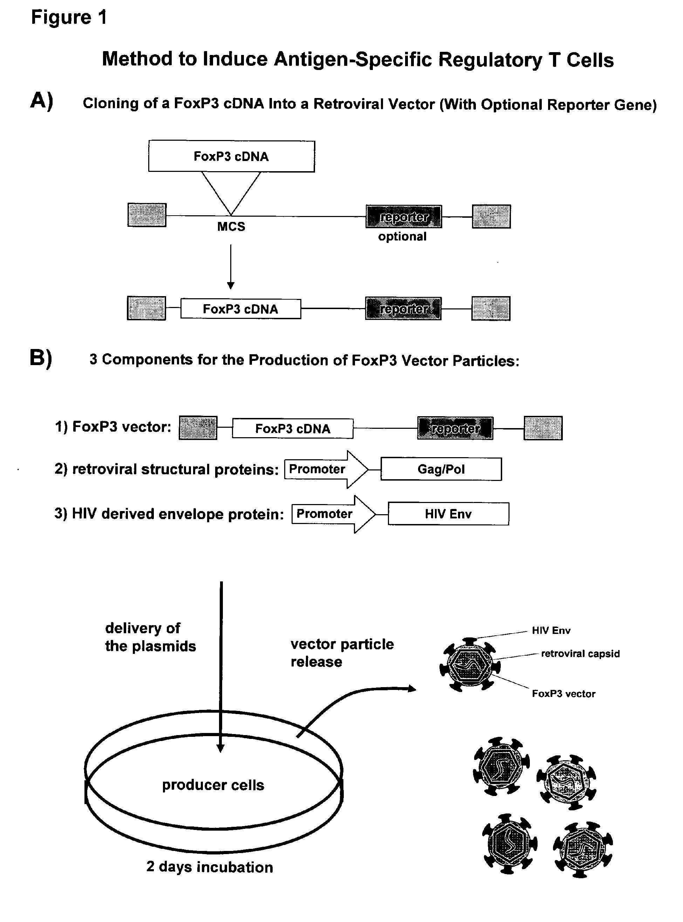

Figure 1A is a schematic representation of the insertion of a FoxP3 cDNA cassette into a retroviral vector. The cDNA is ligated into the vector at the multiple cloning site (MCS or polylinker). The resulting retroviral vector expresses the FoxP3 cDNA under control of the MLV LTR promoter (grey boxes flanking the vector). Alternatively, an internal promoter is used to control expression of the FoxP3 cDNA. The shown vector is bicistronic; a reporter gene encoding, for example, Green Fluorescent Protein (GFP), Cyan Fluorescent Protein (CFP), Yellow Fluorescent Protein (YFP) or DsRed, or luciferase genes is co-expressed for easy identification of vector-transduced (transfected) cells.

Figure 1B is a schematic representation of the preparation of infectious particles according to the present invention. Production of retroviral vector particles, for example, is done in a transient transfection system. Three components are necessary: (1) The retroviral vector itself, which transfers the FoxP3 cDNA into the target cells and integrates into the cellular genome. (2) An expression construct (*gag* / *pol*) for the structural and enzymatic components forming the viral core, which packages the retroviral vector. (3) An expression plasmid for the truncated HIV envelope glycoprotein (Env), which is incorporated into the cellular membrane, that is covering the viral core. The Env protein is responsible for attachment to and entry into the target cell. Co-transfection of cells suitable for vector production, such as HEK293T cells or, for *in vivo* and / or therapeutic purposes, human cell lines and autologous cells, with these three plasmids leads to the release of retroviral vector particles, consisting of an MLV core harboring the FoxP3 retroviral vector, which is covered by cellular membrane carrying the HIV Env. These particles are single-round infectious particles, i.e. after infection of a target cell only the vector itself is expressed, there is no *gag* / *pol* or *env* gene to mobilize the vector once it is integrated, meaning that the infectious particle cannot self-replicate.

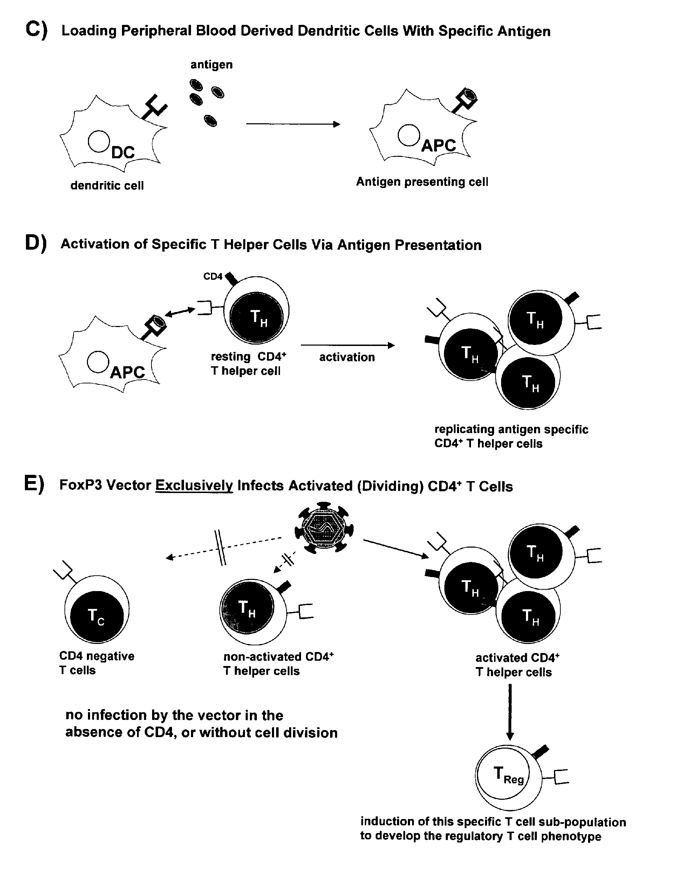

Figure 1C is a schematic representation of the loading of peripheral monocyte-derived dendritic cells (DCs) with a specific antigen. Dendritic cells are cultured in the presence of the desired specific antigen. DCs are professional antigen presenting cells (APCs); they take up the antigen very efficiently and present it in processed form on MHC-II molecules to CD4⁺ T cells. Alternatively, B-cells may be used as the antigen-presenting cells.

Figure 1D is a schematic representation of the activation of specific T helper cells (T_{H}s) via antigen presentation. Upon the addition of a resting CD4⁺ T helper cell (T_{H}, grey) to the DC culture, both cell types (T_{H} and APC) come into contact. If a CD4⁺ T helper cell carries a T cell receptor that is specific for the antigen presented on MHC-II by the DC, it recognizes this MHC-II bound antigen and the T helper cell becomes activated. Only CD4⁺ T cells (T_{H}, grey) are activated to form replicating antigen specific CD4⁺ T helper cells (T_{H}, black).

Figure 1E is a schematic representation of the selective infection of activated (dividing) CD4⁺ T helper cells (T_{H}, black) by the infectious particle of the present invention. Infection by the FoxP3 vector occurs only if the T cell carries CD4 and is activated at the same time (T_{H}, black). T cells without CD4 (T_{C}, black) cannot be penetrated by an HIV Env bearing vector particle; T cells that are not dividing (i.e. are not activated, T_{H}, grey) cannot be infected by the MLV vector system. Thus, the infection by the FoxP3 vector is antigen-specific, resulting in antigen-specific regulatory T cells (T_{Reg}s, white) which are induced to form a specific T cell sub-population in order to develop the regulatory T cell phenotype.

### Description of the Preferred Embodiments

The present invention provides an infectious particle. The infectious particle may be based on a virus-like particle (VLP). A VLP comprises one or more viral proteins derived from a structural protein of a virus. Additionally, a VLP may contain all or a selection of the remaining proteins derived from structural, envelope and enzymatic proteins of the parent virus which are associated with the assembly of viral particles and with completion of the viral replication cycle.

In the case of retroviruses, the structural, envelope and enzymatic proteins are encoded by the *gag*, *env* and *pol* genes, respectively. During replication of the virus in infected cells, the Gag precursor protein is processed to form the matrix, capsid and nucleocapsid structural proteins, whilst the Pol precursor protein is processed to form the enzymatic proteins with protease, reverse transcriptase and integrase function. It is generally accepted that this processing occurs once retroviral *pol* gene products are incorporated into the developing virions as part of Gag / Pol fusion. The Gag proteins and Gag / Pol fusion proteins then undergo proteolytic processing by the viral protease, so as to form viral cores containing the six proteins described above, surrounded by the viral envelope obtained during virus budding. For the purposes of the present invention, it was necessary to select an appropriate set of viral envelope and structural proteins which are suitable for the antigen-specific infection of regulatory T cells [J. Virol. (1999) 73, 9632].

An infectious particle according to the present invention is modified so as to have a specific immunogenic activity. Specifically, the infectious particle selectively infects dividing CD4⁺ cells. For this purpose, the infectious particle has a surface displaying a ligand binding to a CD4 receptor. In a preferred embodiment, the surface of the infectious particle comprises an envelope protein, Env, which can selectively bind to a CD4⁺ cell. Binding to a CD4⁺ cell is achieved through one or more ligands attached to the surface of the infectious particle.

Suitable ligands are those capable of binding to CD4. In a preferred embodiment, the ligand is a protein. In a further preferred embodiment, the protein is a CD4-binding epitope. Most preferably, the ligand is the glycoprotein gp120 or a modified form thereof, or any molecule comprising a CD4-binding epitope, such as CD4 small-molecule inhibitors, CD4-antibodies, hybrids of the gp120 class of structural proteins or variants of the gp120 class of structural proteins that exist in naturally-occurring virus populations. In the case of the structural protein gp120 (or modified forms thereof), three copies of this protein are formed into a trimer that is anchored to the viral membrane through non-covalent bonds and via a trimer of the glycoprotein gp41.

HIV receptors which are involved in infection, such as the co-receptors CCR5 and CXCR4, may be used as alternatives to the CD4 receptors for the infectious particle of the present invention. Thus, the infectious particle may selectively infect dividing CCR5⁺ cells or CXCR4⁺ cells through use of one or more ligands attached to the surface of the infectious particle which are capable of binding to these co-receptors. Such ligands include CCR5 or CXCR4 small-molecule inhibitors or antibodies. This mechanism of infection is within the scope of the present invention.

A preferred structural protein is a viral structural protein. More preferably, one or more structural proteins comprises a viral structural protein. In a further preferred embodiment, one or more structural proteins comprises a retroviral structural protein. In a preferred embodiment, one or more structural proteins comprises a viral structural protein capable of specifically infecting dividing cells. Thus, the infectious particle of the present invention is based on a proteinaceous particle comprising a capsid of any virus capable of specifically infecting dividing cells. The term "capsid" as used herein means a particulate body formed by self assembly of a plurality of self-assembling retroviral Gag / Pol protein molecules or a plurality of self assembling protein molecules substantially homologous with Gag / Pol protein molecules. In a preferred embodiment, the one or more structural proteins comprises a Murine Leukemia Virus (MLV) structural protein or a capsid of MLV, since MLV selectively infects dividing cells. Alternatively, the one or more structural proteins comprises a hybrid of a MLV structural protein or a chimaera of MLV structural proteins capable of specifically infecting dividing cells.

Thus, the one or more structural proteins comprises the molecule through which the infectious particle selectively binds to a CD4⁺ cell. Additionally, the one or more structural proteins comprises a capsid which serves to contain a viral vector encoding a Forkhead box protein 3 (FoxP3) or a protein inducing expression of FoxP3 in the CD4⁺ cell, once infected. Furthermore, the structural protein comprises the capsid proteins necessary for infection of the cell.

In order to obtain selective expression of the infectious MLV vector particles, the MLV *gag* / *pol* expression construct was employed. This construct was used for transfection. Accordingly, a viral background comprising the human cytomegalovirus (CMV) promoter was used to form the plasmid pCMV-(M)Gag / Pol expressing the MLV *gag* and *pol* genes as the Gag and Gag-Pol wild-type precursors [J. Virol. (1999) 73, 9632].

Alternatively, a chimeric expression construct containing MLV capsid in the background of HIV (for example, MLV MA, p12 and CA replacing their HIV counterparts) may be used to obtain a packaging system for the production of lentiviral vectors that exclusively infect dividing cells [J. Virol. (2004) 78, 5670 and PLoS Pathogens (2007) 3, e156].

An infectious particle according to the invention comprises a viral vector. The viral vector comprises a gene of interest functional in a CD4⁺ cell, said gene of interest encoding a Forkhead box protein 3 (FoxP3) or a protein inducing expression of Forkhead box protein 3 in the CD4⁺ cell. In a preferred embodiment, the viral vector is derived from a retroviral vector. In a preferred embodiment, the capsid of the infectious particle of the present invention carries a vector, which, when released into the cytoplasm of a CD4⁺ cell, expresses Forkhead box protein 3 or induces expression of Forkhead box protein 3 in the CD4⁺ cell. Thus, the viral vector containing a gene of interest functional in a CD4⁺ cell is functional in so far as it expresses Forkhead box protein 3 or induces expression of Forkhead box protein 3 in the CD4⁺ cell. TGF-β is an example of a factor which induces expression of FoxP3 in CD4⁺ cells.

The gene of interest functional in a CD4⁺ cell comprises a long terminal repeat (LTR) containing an enhancer and a promoter. The gene of interest functional in a CD4⁺ cell is either episomal, wherein it is established autonomously in a CD4⁺ cell, such that it is replicated and transferred independent of the genome of the host, or it is integrated into the genome of the host cell. In a preferred embodiment, the gene is integrated into the host cell genome, thereby resulting in a memory effect, wherein the gene of interest is functional for extended time periods. In a preferred embodiment, the gene of interest is functional in a T_{Reg} cell for up to 1 week. More preferably, the gene of interest is functional for a period of between 1 week and 10 years, most preferably for the lifetime of the cell and for subsequent generations of cells derived therefrom.

The infectious particle thus comprises the molecule through which the infectious particle selectively binds to a CD4⁺ cell, the capsid proteins which are necessary for infection of the cell, and the viral vector encoding a Forkhead box protein 3 (FoxP3) or a protein inducing expression of Forkhead box protein 3 in the CD4⁺ cell. The infectious particle is, thus, an artificial self-inactivating retrovirus, incapable of self-replicating upon infection of cells, for the purposes of safety to the patient.

The encapsulating capsid carrying the vector is preferably enveloped by a HIV envelope protein, Env, comprising one or more gp120 ligands. In a further preferred embodiment, the selective infection of the dividing CD4⁺ cells by the infectious particle is mediated by an HIV envelope.

The infectious particle of the present invention may be used for producing antigen-specific regulatory T cells, T_{Reg}s. Antigen-specific regulatory T cells are a specific sub-population of T cells involved in regulation of an immune response against a specific antigen. Such T cells may therefore be used in regulation of a specific immune response against a specific antigen.

The invention provides a process for producing antigen-specific regulatory T cells. In a preferred embodiment, the process for producing antigen-specific regulatory T cells is an *in vitro* process. The process for producing antigen-specific regulatory T cells *in vitro* comprises
(i) contacting antigen-presenting cells (APCs) with an antigen reactive with the antigen-presenting cells for providing antigen-presenting cells complexed with an antigen, (ii) activation of T cells with the antigen-presenting cells complexed with an antigen for providing activated T cells, and (iii) infecting the activated T cells with an infectious particle according to the invention for expressing or inducing expression of Forkhead box protein 3 so as to prepare an antigen-specific regulatory T cell. In other words, the process for producing antigen-specific regulatory T cells using the infectious particle of the present invention is performed *ex vivo,* whilst the antigen-specific regulatory T cells, thus produced, are suitable for therapeutic, *in vivo* use.

The complex of the antigen and antigen-presenting cells involves reaction of an antigen with the antigen-presenting cells. In a preferred embodiment of the invention, the antigen-presenting cells (APCs) are dendritic cells, DC and the antigen-presenting cells complexed with an antigen are dendritic cells complexed with an antigen. In a further embodiment of the invention, dendritic cells are reacted with an antigen to provide antigen-presenting cells complexed with an antigen. In a further embodiment, the antigen-presenting cells complexed with an antigen are used to activate T cells, thereby providing activated T cells. Activated T cells are T cells which are undergoing division. In a further embodiment, the activated T cells are infected with an infectious particle according to the invention. The process of infection of the activated T cells is directed to expressing or inducing expression of Forkhead box protein 3 in said T cells. The expressing or inducing expression of FoxP3 in activated T cells results in preparation of antigen-specific regulatory T cells.

Whilst the present invention involves a three-step process for the production of T cells (as described above), it is, however, recognised that this same result may be achieved by an alternative three-step process. This alternative process involves (i) isolation of T cells, (ii) infection of the sub-population of all actively dividing T cells in a manner that is non-specific for CD4⁺ cells and (iii) isolation and purification of the antigen-specific cells using a matrix-isolation or matrix-purification method such as column chromatography, whereby the matrix carries a major histocompatibility complex, or parts thereof, which presents a peptide specific for the given antigen. This latter method is extremely costly and time-consuming, as well as being restricted to particular peptides available for use in the isolation process.

The present invention also provides a process for producing an infectious particle according to any one of claims 1 to 10, comprising an *in vitro* step of contacting one or more structural proteins with a viral vector containing a gene of interest functional in a CD4⁺ cell, said gene of interest encoding a Forkhead box protein 3 or a protein inducing expression of Forkhead box protein 3 in the CD4⁺ cell.

The present invention also provides a composition comprising the infectious particle and a carrier. Further, the invention provides a pharmaceutical composition comprising a plurality of particles according to the invention, together with a pharmaceutically acceptable carrier.

The present invention also provides a kit of parts. The kit of parts is for antigen-specific regulatory T cell induction. Antigen-specific regulatory T cell induction is the process of inducing the production of T cells involved in the regulation of an immune response against a specific antigen. The kit of parts comprises a virus-like particle selectively infecting CD4⁺ cells as defined by the infectious particle according to the invention and one or more antigens.

The antigen is an antigen of choice selected based on which antigen it is desired that the regulatory T cell controls an immune response against. Suitable antigen sources include, but are not restricted to, proteins, protein extracts, DNA and RNA from major histocompatibility complexes, tumors, viruses and other pathogens, and viral vectors. The antigens used in the present invention are selected from a list which includes, but is not restricted to, ganglioside GM1 (Guillan Barré Syndrome), myelin basic protein (Multiple Sclerosis), myelin oligodendrocyte glycoprotein (Multiple Sclerosis) and transplantation antigens such as major histocompatibility complexes (MHCs) or antigens of the ABO blood group system, either singly or in combination.

While it is generally problematic to introduce foreign genes into a human chromosome, for example, by retroviral transduction, it is to be acknowledged that constant development of novel retroviral delivery systems have improved their safety significantly. Current retroviral vectors integrate non-specifically within areas of active transcription. This guarantees expression of the retrovirally-transferred gene(s) but, at the same time, bears the risk of insertional mutagenesis or the activation or over-expression of otherwise tightly-controlled genes (*i.e*. proto-oncogenes), thus leading to a risk of tumorigenesis. To reduce such a risk, retroviral vectors have been modified for example, by removal of the retroviral enhancer sequences, and the use of cellular promoters driving the transgene. Ongoing development in the field constantly leads to further improvement of safety issues. For example, many laboratories are currently involved in development of retroviral vectors that integrate specifically in a desired chromosomal region, thereby minimizing tumorigenic effects. These and other developments may render such vector systems safe vehicles for FoxP3, however the extent of their safety and applicability is not yet fathomable. In the present invention, patients are not immune-suppressed: their immune system is fully functional. Furthermore, our method enables tests for integration in individual T cell clones *in vitro*, before appplication in the patient.

The present invention also provides a T cell infected with a retroviral vector containing a gene of interest functional in a CD4⁺ cell, which encodes a Forkhead box protein 3, homologs of FoxP3 which retain the function of FoxP3, or a factor inducing expression of FoxP3. In a preferred embodiment, the gene of interest functional in a CD4⁺ cell encodes a Forkhead box protein 3 or a protein inducing expression of Forkhead box protein 3.

CD4 (cluster of differentiation 4) is an immunoglobulin glycoprotein expressed on the surface of monocytes, macrophages and T_{H}, T_{Reg} and dendritic cells. CD4 is the co-receptor on T cells for the T cell receptor (TCR). T cells expressing CD4 molecules are specific for antigens presented by MHC-II, since CD4 uses one of its four immunoglobulin domains, D1, to interact with the β₂-domain of MHC-II molecules. Furthermore, CD4 is a primary receptor which HIV uses to gain entry into host cells. HIV binds to CD4 via its envelope protein, in particular via the protein gp120. Binding of CD4 to this protein changes its conformation, such that HIV can bind to two additional host cell surface receptors and fuse its outer viral membrane with that of the host cell.

CD4⁺ T cells (T cells expressing the CD4 cluster) were isolated from donor blood samples according to standard procedures, most preferably using the commercial MACS^{®} cell separation system available from Miltenyi Biotec. This system is based on a technique in which cells expressing CD4 are labelled with CD4-specific antibodies bound to MACS^{®} microbeads containing small amounts of magnetic material. Loading of the mixture of labelled and unlabelled cells upon a MACS^{®} column which is placed in or provides a magnetic field, followed by subsequent elution, results in retention of those cells which are labelled. The labelled CD4⁺ T cells may be subsequently eluted upon removal of the magnetic field.

Media suitable for the growth or maintenance of cell cultures include, but are not restricted to, Dulbecco's Modified Eagle's Medium (DMEM), Iscove's modified Dulbecco's Modified Eagle's Medium, McCoy's 5A medium, EHAA 120 medium or phenol red-free RPMI 1640 medium. For the culture of T cells, the use of RPMI 1640 medium is most preferred.

The media may optionally be supplemented with additives which are selected from a list which includes, but is not restricted to, glucose (at concentrations of from 0.1 to 2.0 mg/mL), L-glutamine (at concentrations of from 1 to 5 mmol/L), penicillin (at from 1 to 1000 U/mL), streptomycin (at concentrations of from 1 to 1000 µg/mL), puromycin (at concentrations of from 0.1 to 30 µg/mL), G418 (at concentrations of from 0.1 to 2.0 mg/mL), serum [including fetal calf serum (FCS) or horse serum (at concentrations of from 1 to 30 %, most preferably 10%)], HEPES (at concentrations of from 1 to 100 mmol/L), β-mercaptoethanol (at concentrations of from 1 to 500 µmol/L) or sodium pyruvate (at concentrations of from 0.01 to 100 mmol/L), either singly or in combination. For the culture of T cells, the combination of 0.4 mg/mL glucose, 10 to 20 % FCS, 300 µg/mL L-glutamine, 100 U/mL penicillin and 100 µg/mL streptomycin, when used with RPMI 1640 medium, is most preferred.

For therapeutic applications in humans, the use of serum-free media is preferred. Such media formulations are available from Invitrogen, CellGenix, BD Biosciences, Biochrom and Hyclone, amongst others.

Culture of cells was performed using any of the above culture media, optionally containing the above additives, whilst maintaining the given medium at between 35 and 39 °C, preferably 37 °C, and at 85 to 99% humidity, preferably 95% humidity, under an aerobic atmosphere containing CO₂ (in concentrations of between 1 and 20%, preferably at a concentration of 5%). Alternatively, culture of cells was performed as above, under an aerobic atmosphere using CO₂-independent media formulations. Preferably, an incubator was used to maintain constant temperature, humidity and CO₂ concentrations in the culture media.

Transformation of cells was performed using standard methods of transfection in which cells suitable for vector production, such as HEK293T cells or, for *in vivo* and / or therapeutic purposes, human cell lines and autologous cells, were cultured or maintained in a suitable medium and treated with the constructs encoding structural viral proteins, viral envelope proteins and the FoxP3 retroviral vector. After treatment with the constructs, the cell culture was either immediately subjected to transfection or was further incubated prior to transfection in order to facilitate the uptake of DNA.

Transfection may be performed using any standard method for the uptake of DNA. Standard methods of transfection may be selected from a list which includes, but is not restricted to, chemical shock treatment, chemical treatment, magnetofection or electrotransfection (electroporation), either singly or in combination.

Transfection by chemical shock treatment includes, but is not restricted to, methods of calcium phosphate precipitation or polyethylene glycol treatment using PEG as a 40% aqueous solution.

Transfection by chemical treatment includes methods using chemicals and materials. Chemicals used to effect transfection by chemical treatment comprise proprietary transfection reagents, which may be selected from a list which includes, but is not limited to, Lipofectamine, Dojindo Hilymax, jetPEI, DreamFect, Effectene and Fugene, either singly or in combination. Materials used to effect transfection by chemical treatment involving retention of the DNA on the surface of the material or by encapsulating it include, but are not restricted to, metals such as gold, polymers, nanoparticles, liposomes and DEAE-Dextran, either singly or in combination. In the case of encapsulation, liposomes composed of cationic lipids and highly-branched organic dendrimers find particular application.

Electrotransfection (electroporation) is performed using AC electric fields (radiofrequency transfection; using radiofrequencies in the range of 1 to 500 kHz, electric fields of from 0.01 to 2.0 kV/cm, multiplicity of pulses of from 1 to 10, pulse width of from 1 to 5 ms) or DC electric fields (field of from 0.01 to 2.0 kV/cm, single pulse, time constant of exponential decay of from 0.001 to 10 ms).

These methods increase the rate and degree by which transformation (transfection) occurs by increasing the porosity of the membrane, particularly through the disruption of function in transmembrane proteins (such as Na, K-ATPases). The most preferred method of transfection is that of calcium phosphate precipitation.

Transient transfection of cells with the constructs (1), (2) and (3) effects formation of retroviral vectors: the ability of MLV vector particles to incorporate heterologous envelope proteins means that the resulting retroviral vector particles have the same infection specificity for CD4⁺ cells as does HIV.

The enhanced green fluorescent protein, eGFP, was used as a reporter molecule suitable for monitoring transduction based on gene expression. Detection of a fluorescence signal of eGFP by flow cytometry is not only evidence that the vector containing the eGFP gene, and any other ligated DNA, has been expressed, but also that transduction has occurred. The eGFP is useful as a reporter molecule because it emits a readily detectable fluorescence signal and exhibits minimal cytotoxicity. In the present invention, transduction of the retroviral vector importantly encoding the FoxP3 transcription factor was monitored by ligating the FoxP3 cDNA into the Moloney Murine Leukemia virus-based (MLV-based) retroviral vector backbone, pLZRS-eGFP [Hum. Gene Ther. (1999) 10, 5]. Ligation was performed using Bam HI and Not I restriction digests, thereby resulting in the retroviral vector pLZRS-eGFP-FoxP3. FoxP3 expression was, thus, controlled by the retroviral LTR and its presence in infected cells was monitored through the eGFP expression from the same bicistronic mRNA, mediated by the internal ribosomal entry site [IRES; Hum. Gene Ther. (1999) 10, 5].

Alternatively, any other suitable reporter genes, such as those encoding Cyan Fluorescent Protein (CFP), Yellow Fluorescent Protein (YFP) or DsRed, or luciferase genes were used to monitor FoxP3 expression.

To obtain selective expression of the MLV vector particles, the MLV *gag* / *pol* expression construct was used. This construct was used for transfection once pseudotyped into a suitable viral plasmid background. Accordingly, a viral background comprising the human cytomegalovirus (CMV) promoter was used to form the plasmid pCMV-(M)Gag / Pol expressing the MLV *gag* and *pol* genes as the Gag and Gag-Pol wild-type precursors. The introduction of this plasmid into cells results in the production of MLV VLP cores. Only when the plasmid is co-expressed with an amphotropic MLV envelope (or a related envelope protein) and MLV vector, is an infectious virus produced.

To obtain selective expression of the HIV envelope protein, Env, the *env* expression construct was used. The production of retroviral vectors by transient transfection has been previously described [Proc. Natl. Acad. Sci. USA (1993) 90, 8392] as has the ability of MLV particles to incorporate heterologous envelope proteins [J. Virol. (1998) 72, 7685; J. Virol. (2004) 78, 12537; J. Virol. (1991) 65, 1202; Hum. Gene Ther. (1996) 7, 913]. In order to successfully incorporate HIV envelope proteins into MLV vector particles, they have to be truncated C-terminally.

An expression construct, HXBH10-*gag*⁻ / ΔCT, for the formation of a C-terminally truncated Env protein of HIV was generated. The resulting construct, HXBH10-*gag*⁻ /ΔCT, is a modified variant of the mutant HIV-1 proviral clone HXBH10-*gag*⁻ that produces wild type HIV envelope proteins and differs from the latter in that codon 713 of the *env* gene is replaced with a premature termination codon (TAA). In addition to the C-terminally-truncated transmembrane protein, this construct encodes the surface glycoprotein gp120. This construct was combined with an HIV long terminal repeat thereby rendering it functional as an expression construct for the C-terminally truncated Env protein [J. Virol. (1997) 71, 3341].

An alternative expression construct, pLβAc / env-Tr712-neo, for the formation of a C-terminally truncated Env protein of HIV was generated [Proc. Natl. Acad. Sci. USA (1997) 94, 8640]. The resulting construct, pLβAc / env-Tr712-neo, comprises a variant HIV *env* gene (derived from pTr712) and a neomycin resistance gene which, upon expression, result in formation of a surface glycoprotein gp120 and a variant of the trans-membrane protein of HIV-1 which lacks 144 amino acids at the carboxyl-terminus, relative to the native protein.

Alternatively, a truncated Env under transcriptional control of the HIV promoter was constructed.

In order to generate antigen-presenting cells suitable for presenting a selected antigen to the T helper cells, dendritic cells derived from peripheral blood monocytes (peripheral blood nuclear cells, PBMC) were prepared. The procedure for the preparation of the antigen-presenting dendritic cells involves the three steps of (i), isolation of monocytes; (ii), differentiation of the monocytes to dendritic cells; and (iii), loading the dendritic cells with an antigen of choice to thereby form antigen-presenting cells complexed with an antigen.

Peripheral blood monocytes were isolated from the same blood samples as the T cells through standard cell separation methods. Specifically, CD14⁺ monocytes were sourced from Ficoll-Paque (GE Healthcare) preparations of peripheral blood mononuclear cells (PBMCs), followed by purification with either the commercial MACS^{®} cell separation system available from Miltenyi Biotec or a technique involving plating of the monocytes. Alternatively, CD14⁺ cells may be selected by flow cytometry.

In the Ficoll-Paque flotation procedure, monocytes are separated from other elements in blood using density-gradient centrifugation. A sample of blood is lowered onto a Ficoll-sodium metrizoate gradient of specific density and, following centrifugation, monocytes are collected from the plasma-Ficoll interface.

In the commercial MACS^{®} cell separation system available from Miltenyi Biotec, PMBCs expressing CD14 were labelled with a CD14-specific antibody bound to MACS' microbeads, whilst maintaining the monocytes in RPMI 1640 medium supplemented with FCS, penicillin and streptomycin. Loading of the mixture of labelled and unlabelled cells upon a MACS^{®} column, followed by subsequent elution in the presence of a magnetic field, resulted in retention of the labelled cells. The labelled CD14⁺ cells were subsequently eluted upon removal of the magnetic field.

Alternatively, PBMCs were plated in a tissue culture flask, optionally containing beads. CD14-specific antibody was bound to an internal surface or surfaces of the flask, and / or beads, thereby permitting the selective adherence of CD14⁺ monocytes to the said surface. Treatment of the said surface or surfaces of the tissue culture flask and or beads housed therein with PBMCs and subsequent removal of non-adherent cells resulted in selective isolation of CD14⁺ cells bound to the surface. The tissue culture flask and beads are made of a material or materials suitable for a liquid tissue culture medium. In a preferred embodiment, the tissue culture flask and / or beads are made of a material suitable for binding an antibody. The material is selected from a list which includes, but is not limited to, glass (such as Pyrex^{®}), plastic, resin (such as carboxylate-modified TransFluorSpheres, Affi-gel 10 resin, Reacti-Gel agarose) and metals, either singly or in combination. Most preferably, the flask is made of resin and is bound with the CD14-antibody.

The separated monocytes were initially differentiated into immature dendritic cells (iDCs) upon treatment with interleukin-4 (IL-4) and Granulocyte-macrophage colony stimulating factor (GM-CSF). The resulting iDCs were further differentiated into mature DCs by treatment with tumor necrosis factor alpha.

Differentiation of the monocytes to DCs was tested according to standard procedures using antibodies specific for markers on the monocytes and DCs. These antibodies allowed the differentiation status of the cells to be examined.

For the detection of monocytes, antibodies specific for the CD14 marker were used. CD14 is a glycosyl-phosphatidylinositol-anchored cell surface molecule, which functions as a receptor for lipopolysaccharide (LPS). It is preferentially expressed on human monocytes and macrophages, thereby rendering it useful for the detection of these cells in the isolation and loading of dendritic cells with antigen.

For the detection of DCs, monoclonal antibodies which were raised against a range of markers highly expressed by DCs were used. These antibodies include, but are not restricted to, antibodies available from BD Biosciences, Santa Cruz Biotechnology or R&D Systems, which are effective against the following markers: HLA-DR, ICAM-1 and MHC-I [Stem Cells (1996) 14, 376], CD11b [J. Exp. Med. (2003) 198, 185], CD11 c [Immun. (1994) 82, 487], DC-SIGN [Cell (2000) 100, 575], LFA-1 [Trends Immunol. (2001) 22, 457], or CD86 [Nat. Med. (2004) 10, 475], either singly or in combination.

The additional use of secondary antibodies specific for the Fc region of the respective primary antibody allowed the expression of said markers to be quantified by flow cytometry. Furthermore, the use of secondary antibodies meant that the stained dendritic cells were able to be sorted into distinct cell populations depending on whether or not said cells had differentiated. The secondary antibodies were either fluorescent antibodies or biotinylated antibodies. In a preferred embodiment, the secondary antibodies were fluorescent antibodies. Alternatively, labeled primary antibodies were used. These primary antibodies include, but are not restricted to, fluorescein-labelled antibodies, such as FITC-anti-HLA-DR, and PE-labelled cocktail (CD3, CD14, CD19 and CD56). Fluorescence activated cell sorting (FACS) was performed on samples of the labelled DCs to which an anti-aggregation additive had been added, using a FACSCalibur cell sorter in conjunction with CellQuest software (available from Becton-Dickinson & Co).

Cell sorting was performed using a fluorescence activated cell sorter selected from a list which includes, but is not limted to FACSCalibur and FACS Vantage SE / DIVA high-performance cell sorters used in conjunction with CellQuest software (available from Becton-Dickinson).

Following differentiation and isolation, DCs were loaded with the antigen of choice (Figure 1 C) through its addition to the culture medium. The methods of loading or "pulsing" by which DCs of the present invention are loaded with antigen include, but are not restricted to, the use of protein delivery, DNA and RNA delivery and recombinant viral vector delivery, either singly or in combination.

Protein delivery methods include, but are not restricted to, the use of peptide conjugates (conjugation of full-length proteins into transporter peptides to facilitate translocation across the DC membrane and into the MHC-II pathway), the use of fusion constructs (such as HIV *tat*) and the use of lysosomal encapsulation of a specific protein.

DNA and RNA delivery methods include, but are not restricted to, the methods of transfection (detailed above).

Recombinant viral vector methods include use of said vector expressing an antigen to deliver transgene products into DCs.

The culture media used for *in vitro* loading and generating of DCs is, optionally, supplemented with supplements which include, but are not restricted to, cytokines, Tumor Necrosis Factor-α (TNF-α), CD40 ligand, interleukin-12 and interleukin-2, either singly or in combination.

The dendritic cells according to the invention are, alternatively, replaced with B cells and contacted with an antigen in order to provide antigen-presenting cells complexed with an antigen. The B cells used in the process of producing antigen specific regulatory T cells are B lymphocytes. The B cells are derived from immature B cells isolable from bone marrow. In an embodiment of the invention, the B cells are contacted with an antigen according to the methods described herein for DCs. The antigen is reactive with the B cells, thereby providing antigen-presenting cells complexed with an antigen.

In an embodiment of the invention, the antigen-presenting cells complexed with an antigen are used to activate T helper cells, T_{H}, thereby providing activated T cells. Activated T cells are T cells which are undergoing division. Thus, once loaded with antigen, the DCs or, optionally, B cells, were co-cultured with T helper cells, T_{H} (Figure 1D). In this setting, the T_{H} cells were able to scan via their T cell receptor (TCR) the antigen presented on the major histocompatibility complexes (MHC) on the antigen-presenting cells. Once a TCR found the cognate antigen / MHC, the respective CD4⁺ T_{H} cell was activated, thereby resulting in the formation of an antigen specific CD4⁺ T_{H} cell which was actively dividing. In other words, a sub-population of CD4⁺ T_{H} cells specific for a given antigen were formed. This is a strictly specific process, ensuring antigen specific immune responses [Annu. Rev. Immunol. (2002) 20, 621]. Once activated, the antigen specific T cells were susceptible to infection by the MLV derived vector particles.

In a further embodiment, the activated T cells are infected with an infectious particle according to the invention. The process of infection involves the transduction of the activated host T cells by the infectious particle according to the invention. The process of infection is directed to expressing or inducing expression of Forkhead box protein 3 in said T cells. The expressing or inducing expression of FoxP3 in activated T cells results in preparation of antigen-specific regulatory T cells.

Due to the intrinsic block of MLV infection in resting (non-dividing) cells, only those cells that were actively dividing were infected by the MLV-based retroviral vector particles, thereby excluding all resting T cells. Likewise, vector particles based on a capsid of any virus capable of specifically infecting dividing cells also resulted in selective infection of actively dividing T cells. At the same time, only cells expressing CD4 (CD4⁺ cells) were infected by the HIV envelope-carrying vectors. Accordingly, this limited the infection spectrum of the vector particles of the present invention to those cells that were CD4⁺ and actively dividing, i.e. activated CD4⁺ T cells. Since the cells were activated by a specific antigen, only that subset of CD4⁺ T cells which is specific for the said antigen were observed to undergo cell division.

The infection of antigen-specific CD4⁺ T_{H} cells (Figure 1 E), was performed by challenging the DC / T cell co-culture (or, optionally, B cell / T cell co-culture) containing activated T_{H}, with 0.1 to 10 multiplicities of infection (m.o.i.) of the infectious particle. Infection may be performed by several different methods selected from a list which includes, but is not limited to, static infection, spin-fection and flow-through infection, either singly or in combination.

Static infection was performed by mere addition of the infectious particle to cell cultures, followed by incubation for 24 hours, addition of 500 µL of fresh inoculum and a further 24 hours of incubation.

Spin-infection was performed by centrifugation of a suspension of the cells at 800 G and 25 °C for 1 hour, in the presence of the infectious particle. The supernatant was removed, fresh inoculum of infectious particle was added and centrifugation was again performed.

Flow-through infection was performed using a membrane of small pore size (0.4 µm) to retain cells whilst permitting the supernatant containing the infectious particle to flow through. The addition of fresh inoculum of infectious particle and flow through infection was perfomed on the cells, thus retained.

One necessary control in this setting was the challenge with the infectious particle in the presence of T cell stimulators. Chemical stimulators suitable for the purposes of the present invention include, but are not restricted to concanavalin A, phytohemagglutinin, phorbol myristate acetate, ionomycin, anti-CD3 and anti-CD28, either singly or in combination. The chemical stimulators were added to the cells infected with VLP in concentrations of between 1 ng / mL and 10 mg / mL. The chemical stimulators were added to the cells between 1 and 6 hours prior to harvest. In a preferred embodiment, 1 µmol/L ionomycin and 10 ng/mL of phorbol myristate acetate were added to the infected cells 4 hours before harvest. These stimulators indiscriminately activated the T cells to divide, which in turn made CD4⁺ T cells with many different antigen specificities available for infection. This is in stark contrast to the specific infection of only those CD4⁺ T cells that are specific for the antigen given to and presented by the DC.

Expression of FoxP3 was analysed by flow cytometry using the internal ribosomal entry site-driven (IRES-driven) reporter gene green fluorescent protein (GFP). Direct measurement was performed using intracellular staining with FoxP3 antibodies (available from Miltenyi Biotec or Becton-Dickinson, amongst others) and quantification was performed by flow cytometry.

There is limited knowledge regarding specific downstream effects of FoxP3 since several hundred genes are bound by FoxP3, with both activating and suppressing effects [Nature (2007) 445, 936]. Some known genes such as G protein coupled receptor 83, extracellular matrix protein 1, CKLF-like MARVEL transmembrane domain containing 7, natural killer cell group 7 sequence, suppressor of cytokine signaling 2 and glutaredoxin, are upregulated but few are FoxP3 dependent [Trends Mol. Med. (2007) 13, 3]. One FoxP3 dependent, upregulated gene is that encoding the lectin, galactoside-binding, soluble 3 (LSGALS3) marker. Thus, the upregulated expression of the FoxP3 dependent gene for the LSGALS3 marker was quantified using standard flow cytometric methods.

The presence of T_{Reg} was confirmed using specific cell surface CD25 and GITR markers and intracellular CTLA-4 markers that are associated with the T_{Reg} phenotype. Specific antibodies for all of these markers exist, are in use and are commercially available. Expression of the said markers was quantified using standard flow cytometric methods. Cytokine production (IL-2, IL-4, IL-10, and IFNγ secretion) was assessed in the T_{Reg} cell co-culture preparation by intracellular staining and analysis using flow cytometry.

One activity of the T_{Reg} phenotype is the suppression of T cell activation. The suppression of T cell activation was tested using co-cultures of T_{Reg} and stimulated T cells to measure the proliferation rate of stimulated T cells. The proliferation rate was measured by standard cell-based immunologic monitoring with CD4⁺ proliferation assays.

Another hallmark of the suppressive effect was the expected reduction of IL-2 production in the T cells stimulated by the addition of T_{Reg}. Concentrations of IL-2 were analyzed using an ELISA protocol. The ELISA protocol was selected from a list of protocols which include, but are not restricted to, those of BD Biosciences, Bender MedSystems and PromoCell.

The present invention will now be illustrated by the following Examples.

### Method of the Invention: Examples Example 1: FoxP3 transferring infectious particles

For the targeted infection of CD4⁺ T cells, an MLV vector system is pseudotyped with heterologous envelope proteins. More specifically, the HIV region encoding the HIV envelope protein (HIV Env) is substituted for that of the MLV so that the MLV vector particles embody an HIV envelope. Retroviral vector particles are produced by transient transfection of human embryonic kidney cells, namely HEK293T cells stably expressing LZRNL with plasmids encoding (1) the FoxP3 expressing retroviral vector, (2) the structural and enzymatic proteins Gag/Pol of murine leukemia virus and (3) a C-terminally truncated envelope protein (Env) of human immunodeficiency virus (HIV).

### (1) FoxP3 expressing retroviral vector

The human FoxP3 sequence is accessible from the Genbank file NM_014009. To obtain FoxP3 cDNA, CD4⁺ T cells were first isolated from donor blood samples according to standard procedures, most preferably using the commercial MACS^{®} cell separation system available from Miltenyi Biotec. Thus, cells expressing CD4 (CD4⁺ T cells) were labelled with CD4-specific antibodies bound to MACS^{®} microbeads. Loading of the mixture of labelled and unlabelled cells upon a MACS^{®} column, followed by subsequent elution in the presence of a magnetic field, resulted in retention of the labelled cells. The labelled CD4⁺ T cells were subsequently eluted upon removal of the magnetic field.

Cellular RNA was extracted from the purified CD4⁺ T cells using standard isolation methods, preferably using the guanidine thiocyanate method, followed by two cycles of oligo(dT)-cellulose column chromatography. The cellular RNA, thus purified, was subsequently used as a template for the cloning of complementary DNA (cDNA) which was used to transfer the FoxP3 cDNA into a retroviral vector. As such, standard procedures of reverse transcription followed by the polymerase chain reaction (RT-PCR) were performed using the isolated cellular RNA as a template, in combination with the primer pair hF1: TCAGGATCCACAAGGACCCGATGCCCAAC and hF2: TATGCGGCCGCTGTTCGTCCATCCTCCTTTC to amplify the FoxP3 encoding region. The PCR product was flanked by primer-introduced unique restriction sites Bam HI at the 5' end and Not I at the 3' end. The FoxP3 cDNA, thus obtained, was ligated into the Moloney murine leukemia virus (MLV) based retroviral vector backbone pLZRS-eGFP using Bam HI and Not I restriction digests, subsequent to the vector's restriction with Bam HI and Not I, thereby resulting in the retroviral vector pLZRS-eGFP-FoxP3 (refer to Figure 1A). FoxP3 expression was thus controlled by the retroviral LTR and its presence in infected cells was monitored through the eGFP expression from the same bicistronic mRNA, mediated by the internal ribosomal entry site. In order to offer greater flexibility with respect to its application, the FoxP3 cassette has also been transferred into further retroviral vectors under transcriptional control of cellular promoters, with and without other reporter genes. The latter is important so as not to introduce any foreign gene which would result in an immune response against the retroviral vector-transduced cells.

### (2) Structural and enzymatic proteins Gag/Pol of murine leukemia virus (ML V)

To obtain selective expression of the MLV vector particles, the MLV *gag* / *pol* expression construct was used. This construct was used for transfection. Accordingly, the human cytomegalovirus (CMV) promoter was used to form the plasmid pCMV-(M)Gag / Pol expressing the MLV *gag* and *pol* genes as the Gag and Gag-Pol wild-type precursors [J. Virol. (1999) 73, 9632]. An alternative to using both of the *gag* and *pol* genes in the single construct, is to incorporate these genes into separate vectors, thereby necessitating that both of the plasmids separately expressing Gag and Pol are used for transfection.

### (3) C-terminally truncated envelope protein of human immunodeficiency virus

In order to successfully incorporate HIV envelope proteins into MLV vector particles, they have to be truncated C-terminally. An expression construct, HXBH10*-gag⁻* / ΔCT, for the formation of a C-terminally truncated Env protein of HIV was generated [J. Virol. (1997) 71, 3341]. The resulting construct, HXBH10-*gag⁻* / ΔCT, is a modified variant of the mutant HIV-1 proviral clone HXBH10-*gag*⁻ that produces wild type HIV envelope proteins and differs from the latter in that codon 713 of the *env* gene is replaced with a premature termination codon (TAA). In addition to the C-terminally-truncated Env protein, this construct encodes the surface glycoprotein gp120. This construct was combined with an HIV long terminal repeat thereby rendering it functional as an expression construct for the C-terminally truncated Env protein. As an alternative, a plasmid encoding a truncated HIV envelope under transcriptional control of the CMV promoter (pCDNA3-HIVenvdel712) was used to pseudotype the MLV vector.

For the targeted infection of CD4-expressing cells, the MLV vector system was pseudotyped with the truncated transmembrane protein and the surface glycoprotein gp120 of HIV.

Transfection of human embryonic kidney cells (HEK293T cells) was performed using calcium phosphate precipitation in which cells were cultured or maintained in Dulbecco's Modified Eagle's Medium (DMEM, Invitrogen) supplemented with 0.4 mg/mL glucose, 10 to 20 % FCS, 2 mM L-glutamine, 100 U/mL penicillin and 100 µg/mL streptomycin (Invitrogen) at 37 °C, 95 % humidity, and 5 % CO₂. After transient co-transfection (Figure 1 B) of HEK293T cells with the three plasmid constructs (1), (2) and (3), the transfected cells were incubated for 24 hours, after which time, the medium was replaced by fresh culture medium. The following day, the culture supernatant containing the FoxP3-transferring infectious particles (vector particles) was harvested, filtered through 0.45 µm pore sized syringe filters (Millipore), and used to infect the target T cells. For comparison, standard cell lines (e.g. Hut78) were infected in parallel to control the infectious titer of the vector preparation under standardized conditions. Diluted volumes containing the infectious particles were formed so as to generate a 10-fold dilution series suitable for assays. If required, the supernatant obtained during infectious particle formation and / or the diluted volumes were concentrated 100-fold by ultracentrifugation at 80000 to 125000 *g* at 4 °C in an ultracentrifuge through a 20% sucrose cushion, in order to reduce the volumes required to infect cell cultures. This procedure is based on standard protocols, such as that described in J. Virol. (2004) 78, 12537. Aliquots of the infectious particle containing supernatants were stored at -80 °C prior to use.

### Example 2: Isolation of dendritic cells

Monocyte-derived dendritic cells were prepared by induction of monocyte differentiation into dendritic cells (DC). Accordingly, peripheral blood monocytes were isolated from the same blood samples as the T cells through standard cell separation methods. Specifically, CD14⁺ monocytes were sourced from Ficoll-Paque (GE Healthcare) preparations of peripheral blood mononuclear cells (PBMCs), followed by purification with either the commercial MACS^{®} cell separation system available from Miltenyi Biotec or a technique involving plating of the monocytes.

The T-depleted monocytes obtained from the Ficoll-Paque isolation procedure were subjected to treatment with a CD14-specific antibody bound to MACS^{®} microbeads, whilst maintaining the cells in RPMI 1640 medium supplemented with 10 % FCS, 100 U/mL penicillin and 100 µg/mL streptomycin. Loading of the mixture of labelled and unlabelled cells upon a MACS^{®} column, followed by subsequent elution in the presence of a magnetic field, resulted in retention of the labelled cells. The labelled CD14⁺ monocytes were subsequently eluted upon removal of the magnetic field.

After separation of the monocytes, they were treated with 100 ng/mL interleukin-4 (IL-4, available from R&D Systems) and 50 ng/mL Granulocyte-macrophage colony stimulating factor (GM-CSF, available from R&D Systems) for 4 to 6 days. The resulting immature dendritic cells (iDCs) were further differentiated into mature DCs by treatment with tumor necrosis factor alpha.

Differentiation of the monocytes to DCs was tested according to standard procedures using antibodies specific for markers on the monocytes and / or DCs. For the detection of monocytes, antibodies specific for the CD14 marker were used [Immunol. Today (1993) 14, 121]. For the detection of DCs, monoclonal antibodies which were raised against a range of markers highly expressed by DCs were used. Specifically, antibodies raised against the HLA-DR, ICAM-1 and MHC-I [Stem Cells (1996) 14, 376], CD11b [J. Exp. Med. (2003) 198, 185], CD11c [Immun. (1994) 82, 487], DC-SIGN [Cell (2000) 100, 575], LFA-1 [Trends Immunol. (2001) 22, 457] and CD86 [Nat. Med. (2004) 10, 475] markers were used. The antibodies raised against markers expressed by DCs were added to cells in 100 µL of PBS containing 0.1 % sodium azide and 2% FCS. After incubation for 10 to 30 minutes at 25 °C, the stained cells were washed and resuspended in PBS / 2% FCS.

Additionally, secondary fluorescent antibodies specific for markers including fluorescein-labelled FITC-anti-HLA-DR and PE-labelled cocktail (CD3, CD14, CD19 and CD56) were used to quantify the expression of the markers by flow cytometry. Furthermore, the secondary antibodies were used to effect cell sorting. Fluorescence activated cell sorting (FACS) was performed on samples of the labelled DCs using a FACSCalibur cell sorter in conjunction with CellQuest software (available from Becton-Dickinson & Co) [J. Exp. Med. (1993) 178, 1067].

### Antigen loading and maturation of dendritic cells

Following differentiation and FACS, DCs were loaded with the antigen of choice (Figure 1 C) through its addition to the culture medium according to previously described methods [Int. J. Med. Microbiol. (2007) 298, 11; Annu. Rev. Immunol. (2000) 18, 245; J. Exp. Med. (1990) 172, 631; Science (2000) 288, 522]. The antigens chosen for the purposes of the invention include, but are not restricted to, ganglioside GM1 (Guillan Barré Syndrome), myelin basic protein (Multiple Sclerosis), myelin oligodendrocyte glycoprotein (Multiple Sclerosis) and transplantation antigens such as major histocompatibility complexes (MHCs) or antigens of the ABO blood group system. Antigen "pulsing" was used to load DCs of the present invention with antigen and form antigen-presenting cells complexed with an antigen.

Thus, after 6 days of culture in GM-CSF and IL-4, immature DCs were loaded with cell lysate (*e*.*g*. lysate of cells from organ transplants, or antigens) or purified antigen. Once loaded with lysate, immature DCs were cultured for 2 to 3 hours at 37 °C at a concentration of 1 x 10⁶ to 5 x 10⁶ cells/mL. Following antigen loading, cells were pelleted, washed to remove unbound lysate or unbound antigen, and matured for 48 hours. CD3, CD14, CD83 and CD209 cell surface expression was characterized by flow cytometry. At this stage, DCs were cryopreserved or co-cultured with T cells [J. Immunol. Meth. (2003) 277, 1; Vaccine (2006) 24, 3203]. For the loading with purified antigen, DC were incubated with the antigen at a concentration of 20 to 50 µg/mL for 14 to 18 hours. The DC were pelleted, washed and resuspended in maturation medium. Expression of maturation markers was examined in flow cytometry, as described above.

### Example 3: Mixed leukocyte reaction

The medium that was used was the same mixture as described before for T cell culture. T cell-enriched fractions were obtained from PBMC prepared from buffy-coats or leukaphereses. T cells were used at a concentration of 2 x 10⁶ cells/mL, DCs were used at a concentration of 2 x 10⁵ cells/mL. A ratio of 30 : 1 of T cells to DCs and dilution series thereof were performed. T cells without DCs served as a control [J. Immunol. Meth. (2003) 277, 1].

### Example 4: Infection of target T cells

The static infection of T cells (Figure 1 E), was performed by challenging the DC / T cell co-culture containing >95% T_{H}, with 0.1 to 10 multiplicities of infection (m.o.i.) of retroviral vector preparation. The infection procedure used is a standard method employed in many *in vitro* protocols, such as that described in J. Virol. (2004) 78, 12537. Accordingly, the suspension of cells of the DC / T cell co-culture were seeded at 10⁵ cells per well in 24-well plates immediately prior to infection. Infection was performed at 37 °C in a total volume of 500 µL of culture medium optionally containing 10 µg of Polybrene. 24 hours after challenge with the infectious particles, 500 µL of medium was added and a further 48 hours later, the cells were assayed for infection events.

Infection was quantified according to the following Examples 5 and 6.

### Example 5: Examination of FoxP3 expression

Expression of FoxP3 was analysed by flow cytometry according to a previously reported procedure [Science (2003) 299, 1057] using the internal ribosomal entry site-driven (IRES-driven) reporter gene green fluorescent protein (GFP). Direct measurement was performed using intracellular staining with FoxP3 antibodies (available from Becton-Dickinson and others) and quantification in flow cytometry.

### Example 6: Quantification of T_{Reg} markers

The presence of T_{Reg} was confirmed using specific cell surface CD25 and GITR markers and intracellular CTLA-4 markers that are associated with the T_{Reg} phenotype. Specific antibodies for all of these markers exist, are in use and are commercially available from, for example, BD Biosciences, Santa Cruz Biotechnology. Expression of the said markers was quantified using standard flow cytometric methods. Cytokine production (IL-2, IL-4, IL-10, and IFNγ secretion was reduced as a result of the FoxP3 induced T_{Reg} phenotype, for example) was analyzed using the Cytometric Bead Array (Becton-Dickinson) in flow cytometry, as described previously [Science (2003) 299, 1057]. Thus, cytokine production in the harvested DC / T cell co-culture treated with 0.1 to 10 multiplicities of infection (m.o.i.) of retroviral vector preparation (*i.e*. the T_{Reg} cell culture) was assessed by intracellular staining in which cells were re-suspended at 10⁶ / mL. Control cells were stimulated either with 50 ng / mL PMA and 500 ng / mL ionomycin for 4 hours or with 0.5 µg / mL anti-CD3 in the presence of 10⁶ / mL antigen-presenting cells for 24 hours. Two hours prior to harvest, 10 µg / mL of brefeldin A was added. Cells were washed, fixed with 2% paraformaldehyde in PBS for 30 minutes and stained with anti-cytokine or isotype-matched mAbs.

### Example 7: Activity of T_{Reg} as suppressors of T cell responses

The suppression of T cell activation was tested using co-cultures of T_{Reg} and stimulated T cells. The proliferation rate of stimulated T cells was severely reduced in the presence of the T_{Reg}. The proliferation rate was measured by standard cell-based immunologic monitoring with CD4⁺ proliferation assays [BMC Immunol. (2007) 8, 21]. Thus, PBMC were plated in 96-well plates in 24-well replicates at 10⁵ cells / well in media consisting of RPMI 1640 containing L-glutamine, penicillin and / or streptomycin, 2-mercaptoethanol and 10% autologous serum, with or without the addition of the harvested DC / T cell co-culture. After 3 days, the wells were pulsed with 1 µCi of [³H]thymidine for 8 to 10 hours and counted. The stimulation index is defined as the mean CPM of the response of regulated cells divided by the mean of the response of non-regulated cells. Positive and negative controls were run on each plate. Phytohemaglutinin was incubated with T cells as a positive control to assess the ability of the T cells to respond to antigen. Media alone was used as a negative control.

The suppression of T cell activation by the T_{Reg} cells harvested from the DC / T cell co-culture was also identified by a reduction in IL-2 production in the T cells stimulated during co-culture with T_{Reg} (formed as described above). Thus, the concentrations of IL-2 were tested using the ELISA protocol of BD Biosciences (although other protocols such as Bender MedSystems, PromoCell could also be used).

## Claims

1. An infectious particle having a surface displaying a ligand binding to a CD4 receptor and selectively infecting dividing CD4⁺ cells, said particle comprising:
(a) one or more structural proteins, and
(b) a viral vector containing a gene of interest functional in a CD4⁺ cell, said gene of interest encoding a Forkhead box protein 3 or a protein inducing expression of Forkhead box protein 3 in the CD4⁺ cell.

2. The infectious particle according to claim 1, which is a virus-like particle.

3. The infectious particle according to any one of claims 1 or 2, wherein the ligand is a protein.

4. The infectious particle according to claim 3, wherein the protein is a CD4-binding epitope.

5. The infectious particle according to claim 3, wherein the protein is gp120 or a modified form of gp120.

6. The infectious particle according to any one of the preceding claims wherein selective infection of dividing CD4⁺ cells is mediated by an HIV envelope.

7. The infectious particle according to any one of the preceding claims, wherein the one or more structural proteins comprises a viral structural protein.

8. The infectious particle according to claim 7, wherein the one or more viral structural proteins comprises a retroviral structural protein.

9. The infectious particle according to claim 7, wherein the one or more viral structural proteins comprises a murine leukemia virus structural protein.

10. The infectious particle according to any one of the preceding claims wherein the viral vector is derived from a retroviral vector.

11. A composition comprising:
(i) the infectious particle according to any one of claims 1 to 10, and
(ii) a carrier.

12. A process for producing an infectious particle according to any one of claims 1 to 10, comprising an in vitro step of contacting one or more structural proteins with a viral vector containing a gene of interest functional in a CD4⁺ cell, said gene of interest encoding a Forkhead box protein 3 or a protein inducing expression of Forkhead box protein 3 in the CD4⁺ cell.

13. Use of the infectious particle according to any one of claims 1 to 10 for producing antigen-specific regulatory T cells.

14. Process for preparing antigen-specific regulatory T cells in vitro, which comprises:
(i) contacting antigen-presenting cells with an antigen reactive with the antigen-presenting cells for providing antigen-presenting cells complexed with an antigen,
(ii) activation of T cells with the antigen-presenting cells complexed with an antigen for providing activated T cells, and
(iii) infecting the activated T cells with an infectious particle as defined by any one of claims 1 to 10 for expressing or inducing expression of Forkhead box protein 3 so as to prepare an antigen-specific regulatory T cell.

15. The process according to claim 14, wherein the antigen-presenting cells are dendritic cells.

16. Kit of parts for antigen-specific regulatory T cell induction, which comprises a virus-like particle selectively infecting CD4⁺ cells as defined in any one of claims 1 to 10 and one or more antigens.

17. T-cell infected with a retroviral vector containing a gene of interest functional in a CD4⁺ cell, which encodes a Forkhead box protein 3 or a protein inducing expression of Forkhead box protein 3.
